# EUROPEAN PATENT APPLICATION

(11) **EP 4 516 114 A1**
(43) Date of publication of application: **05.03.2025**
(21) Application number: 23386092.3
(22) Date of filing: 09.10.2023
(51) Int. Cl.: A23L 2/38, A23L 2/52, A23L 33/105, A61K 36/25, A23L 33/17, A61K 31/196, A61K 31/197

(54) **FUNCTIONAL NOOTROPIC DRINK**

(30) Priority: 29.08.2023 GR 20230100700
(71) Applicant: Chatzimanolis, Georgios - Konstantinos, 18547 Piraeus (GR)
(72) Inventor: The designation of the inventor has not yet been filed

(57) **Abstract**

This invention is a drink called "i-qube nootropic", whose composition combines specific ingredients in order to improve the mental performance and concentration of the consumer as well as his energy's stimulation.

In our case, we have a drink for everyday use, which can be consumed without restrictions and can also be consumed by any person in order to improve his daily life.

The active composition of this invention consists of eight ingredients, which are theanine, caffeine, tyrosine, choline, rhodiola rosea, gingo biloba, panax ginseng and brahmi (bacopa monnieri). These ingredients have been carefully selected to interact and create a synergy that results in supporting the effect of their overall properties.

## Description

This invention is a drink called "i-qube nootropic", whose composition combines specific ingredients in order to improve the mental performance and concentration of the consumer as well as his energy's stimulation.

Until today, there are no similar products in the field of free-to-eat functional food supplements and drinks advertised as products of such food supplements are consumed under strict conditions and with the aim of the comsumer's health protection and with limitations regarding the quantity, which must be consumed by one person. This means that such conventional products carry the risk of burdening the consumer's health, if he consumes them uncontrollably, and this makes the consumer wary of them.

In our case, we have a drink for everyday use, which can be consumed without restrictions and can also be consumed by any person in order to improve his daily life. It is not a drug and it is not has instructions for use, because its consumption is not subjected to any limits due to its composition. This makes it accessible to more people, who are usually hesitant towards such preparations due to restrictions on their consumption.

The active composition of this invention consists of eight ingredients, which are theanine, caffeine, tyrosine, choline, rhodiola rosea, gingo biloba, panax ginseng and brahmi ( bacopa monnieri). These ingredients have been carefully selected to interact and create a synergy that results in supporting the effect of their overall properties. Each of these ingredients has undergone various treatments for the isolation of their active substances so that this invention will have a palatable drinking effect.

For the preparation of this invention, the following method is followed: initially, the raw materials are gathered i.e. the eight aforementioned components, which can be in the form of powder, plant solution, extract or distillate. Afterwards, we have the processing of the raw materials, each one of them separately, in order to extract their active substances in the form of powder or solution. Techniques such as boiling, pressing, drying, fermentation, extraction or distillation are used to extract these substances. After we finish with the extraction of all the active substances, the rest of the drink ingredients are added together with water in order to create a syrup. This syrup is taken to the bottling plant, where water and carbonate are added, and then to the pasteurization and bottling line.

This invention is available in several colors, depending on the wishes of the consumer.

This invention is available in several flavors.

The active composition of the substances does not affect the color or the taste of this invention.

This invention can be bottled in aluminum cans of various sizes and quantities, in plastic and glass bottles, in nylon packaging and also in bags.

This invention performs the dual function of energy drink and food supplement.

The consumption of this invention contributes to the relief and relaxation of the consumer and helps him to eliminate stress thanks to the theanine ingredient.

This invention increases the conductivity of the nervous system as a result of the caffeine, rhodiola rosea, Asian ginseng and brahmi ingredients.

This invention improves alertness and focus and creates more energy in the body thanks to the caffeine ingredient.

This invention helps fight fatigue and increases the body's resistance to fatigue thanks to the caffeine, rhodiola rosea, brahmi and Asian ginseng ingredients.

This invention contributes to the replenishment of the neurotransmitters norepinephrine and dopamine thanks to its composition, which contains tyrosine.

This invention improves the mental energy of the consumer as a result of its tyrosine and rhodiola rosea ingredients.

This invention enhances the effect of caffeine because tyrosine is on of its components.

This invention improves the mental performance and the consumer's resistance thanks to the theanine and rose rhodiola in its ingredients. This invention enhances the resistance to stress and fatigue due to the presence of rose rhodiola, brahmi and Asian ginseng in its composition.

This invention improves memory and intelligence because its composition includes brahmi, choline and ginkgo biloba.

This invention supports the healthy functioning of the brain thanks to its composition, which includes choline.

This invention improves the blood circulation in the brain thanks to the gingo biloba, which is included in its composition.

This invention supports the memory and the mental well-being thanks to its composition, which contains gingo biloba.

This invention helps the consumer to deal with his mental problems thanks to the gingo biloba included in its composition.

This invention improves the mental performance and the concentration thanks to the Asian ginseng in its composition.

## Claims

1. This invention is a drink called "i-qube nootropic", whose composition combines specific ingredients in order to improve the mental performance and concentration of the consumer as well as his energy's stimulation.

2. Functional nootropic drink according to the Claim 1, which can be consumed without restrictions and can also be consumed by any person in order to improve his daily life.

3. Functional nootropic drink according to the Claim 1, whose ingredients have been carefully selected to interact and create a synergy that results in supporting the effect of their overall properties.

4. Functional nootropic drink according to the Claim 1, whose ingredients have undergone various treatments for the isolation of their active substances so that this invention will have a alatable drinking effect.

5. Functional nootropic drink according to the Claim 1, which can be bottled in aluminum cans of various sizes and quantities, in plastic and glass bottles, in nylon packaging and also in bags.

6. Functional nootropic drink according to the Claim 1, which performs the dual function of energy drink and food supplement.
